# EUROPÄISCHE PATENTANMELDUNG

(11) **EP 0 557 878 A1**
(43) Veröffentlichungstag der Anmeldung: **01.09.1993**
(21) Anmeldenummer: 93102452.5
(22) Anmeldetag: 17.02.1993
(51) Int. Cl.: C07C 205/12, C07C 201/12

(54) **Verfahren zur selektiven Dehalogenierung von ortho-Halogennitrobenzolen**

(30) Priorität: 25.02.1992 DE 4205680
(71) Anmelder: HOECHST AKTIENGESELLSCHAFT, D-65926 Frankfurt am Main (DE)
(72) Erfinder: Kanschik-Conradsen, Andreas, Dr., W-3008 Garbsen 1 (DE); Papenfuhs, Theodor, Dr., W-6000 Frankfurt am Main 50 (DE)

(57) **Zusammenfassung**

Die vorliegende Erfindung bezieht sich auf ein neues Verfahren zur selektiven Dehalogenierung von ortho- Halogennitrobenzolen, wobei Halogen Chlor, Brom oder eine Kombination davon bedeutet, dadurch gekennzeichnet, daß in Nitrobenzolen der allgemeinen Formel (I) 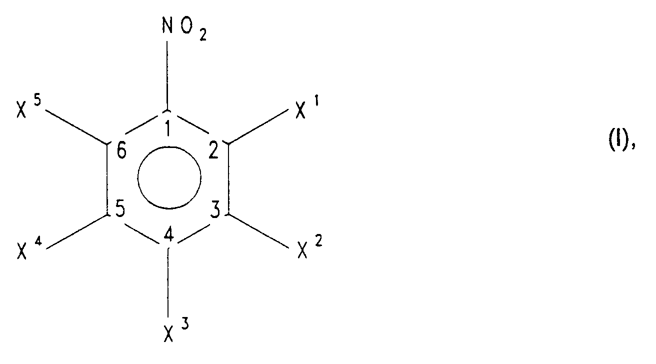
worin X¹ und X³ oder X¹ und X⁵ jeweils Chlor, Brom oder eine Kombination davon bedeuten und die jeweils übrigen Substituenten X unabhängig voneinander Wasserstoff, Fluor oder C₁-C₄-Alkyl bedeuten, durch Umsetzung mit Kupfer in Gegenwart eines Protonendonators die Halogenatome X¹ und X⁵ jeweils durch Wasserstoffatome ersetzt werden.

Von besonderem Interesse ist der Zugang zu dem technisch relevanten 4-Chlor-3-fluornitrobenzol aus 2,4-Dichlor-3-fluornitrobenzol oder 2,4-Dichlor-5-fluornitrobenzol.

## Beschreibung

Die vorliegende Erfindung bezieht sich auf ein neues Verfahren zur selektiven Dehalogenierung von Nitrobenzolen, die in 2- und/oder 6-Stellung zur Nitrogruppe ein oder zwei Chlor- oder Bromatome enthalten.

Der technische Zugang zu 4-Chlor-3-fluornitrobenzol ist mit den bisher üblichen Synthesewegen stark eingeschränkt. So führt beispielsweise die Nitrierung von 2-Fluorchlorbenzol zu einem Gemisch aus 79 % 3-Chlor-4-fluornitrobenzol und nur 21 % 4-Chlor-3-fluornitrobenzol (Houben-Weyl, Methoden der Organischen Chemie, 4. Auflage, Bd. X/1, S. 507). Auch eine Sandmeyer-Reaktion ist aufgrund der aufwendigen Synthese des Ausgangsproduktes 2-Fluor-4-nitroanilin zur Herstellung von 4-Chlor-3-fluornitrobenzol im technischen Maßstab nicht attraktiv.

In der japanischen Patentbekanntmachung Sho-63 156 757 ist ein Verfahren zur selektiven Dehalogenierung von fluorierten ortho-Chlornitrobenzolen mit Hilfe von Natriumborhydrid beschrieben, das jedoch für einen technischen Einsatz zu kostspielig ist.

Es bestand daher ein erhebliches Interesse nach einem neuen ökonomisch sinnvollen Weg zur Herstellung von halogenierten Nitrobenzolen, die in 2- und 6-Position zur Nitrogruppe selektiv dehalogeniert sind, insbesondere zur Herstellung von 4-Chlor-3-fluornitrobenzol, ein Vorprodukt für 3,4-Difluornitrobenzol (DE-A-2 619 792).

Es ist bekannt, daß 2-Chlornitrobenzol, nicht jedoch 3-Chlornitrobenzol und 4-Chlornitrobenzol mit Kupfer in Benzoesäure zu Nitrobenzol reduktiv dechloriert werden kann (W.T. Smith Jr., J. Amer. Chem. Soc., 71, 2852 (1949)). Dieser Autor beschreibt später auch die reduktive Dehalogenierung von 2,5-Dichlornitrobenzol und 2,5-Dibromnitrobenzol in moderaten Ausbeuten von 37 - 38 % zu 3-Chlornitrobenzol bzw. 3-Bromnitrobenzol (W.T. Smith Jr. und L. Campanaro, J. Amer. Chem. Soc., 75, 3602 (1953)). Darüberhinaus weist er nach, daß Monofluornitrobenzole mit Kupfer/Benzoesäure nicht reduktiv defluoriert werden können. Über das Verhalten von Nitrobenzolen, die in 2- und 4-Stellung bzw. in 2- und 6-Stellung zur Nitrogruppe Chlor- oder Bromatome enthalten, war dagegen nichts bekannt. Da sich die elektronischen Verhältnisse im Benzolkern stark verändern, wenn zusätzliche Chlor- oder Bromatome vorhanden sind oder die Position dieser Halogenatome im Ring sich ändert, konnte nicht davon ausgegangen werden, daß sich die Methode von Smith et al. zur Herstellung von 4-Chlor-3-fluornitrobenzol eignete.

Es wurde gefunden, daß überraschenderweise auch 2,4-Dihalogennitrobenzole sowie 2,6-Dihalogennitrobenzole, wobei Halogen Chlor, Brom oder eine Kombination davon bedeutet, mit Kupfer unter Zusatz eines Protonendonators selektiv in der oder den ortho-Position(en) zur Nitrogruppe dehalogeniert werden können.

Darüber hinaus wurde überraschenderweise gefunden, daß durch Zusatz geeigneter Reduktionsmittel die benötigten Mengen an metallischem Kupfer vermindert werden können. Das Potential der Reduktionsmittel muß dabei so hoch sein, daß die bei der Dehalogenierung entstehenden Kupfersalze zu metallischem Kupfer reduziert werden können.

Gegenstand der vorliegenden Erfindung ist ein Verfahren zur selektiven Dehalogenierung von ortho-Halogennitrobenzolen, wobei Halogen Chlor, Brom oder eine Kombination davon bedeutet, dadurch gekennzeichnet, daß in Nitrobenzolen der allgemeinen Formel (I) 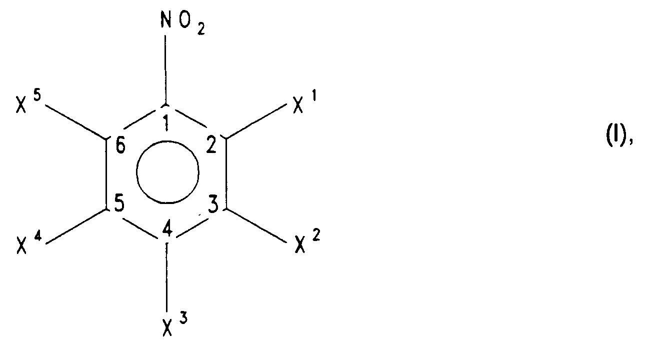
worin X¹ und X³ oder X¹ und X⁵ jeweils Chlor, Brom oder eine Kombination davon bedeuten und die jeweils übrigen Substituenten X unabhängig voneinander Wasserstoff, Fluor oder C₁-C₄-Alkyl bedeuten, durch Umsetzung mit Kupfer, vorzugsweise in Anwesenheit eines Reduktionsmittels, das Kupfersalze zu metallischem Kupfer reduzieren kann, sowie in Gegenwart eines Protonendonators die Halogenatome X¹ und X⁵ jeweils durch Wasserstoffatome ersetzt werden.

Von Interesse sind dabei besonders solche Verbindungen der Formel (I), worin X¹ und X³ oder X¹ und X⁵ jeweils Chlor bedeuten und X² und X⁴ unabhängig voneinander Wasserstoff oder Fluor bedeuten.

Von besonderem Interesse sind die Verbindungen 2,4-Dichlor-3-fluornitrobenzol sowie 2,4-Dichlor-5-fluornitrobenzol, die nach dem erfindungsgemäßen Verfahren selektiv und in guten Ausbeuten in 4-Chlor-3-fluornitrobenzol überführt werden.

Von Interesse ist ebenfalls die Verbindung 2,6-Dichlor-3,5-difluornitrobenzol, die nach dem erfindungsgemäßen Verfahren in 3,5-Difluornitrobenzol überführt wird. Eine Defluorierung findet in keinem Falle statt, ebenfalls unterbleibt eine Dechlorierung in para- oder meta-Position zur Nitrogruppe.

Bei dem erfindungsgemäßen Verfahren kann durch Zusatz geeigneter Reduktionsmittel die benötigte Menge an metallischem Kupfer um bis zu 90 %, vorzugsweise bis zu 60 %, vermindert werden. Als Reduktionsmittel sind alle Stoffe geeignet, deren Redoxpotential ausreicht, die bei der Dehalogenierung entstehenden Kupfersalze zu metallischem Kupfer zu reduzieren. Als Reduktionsmittel im Sinne der Erfindung kommen Aldehyde, reduzierende Zucker, Polyalkohole und Hydrochinone in Betracht. Besonders geeignet sind jedoch aliphatische Aldehyde, insbesondere Methanal, Ethanal, Propanal, Butanal, Methanal oder Ethanal freisetzende Verbindungen wie Trioxan, Paraformaldehyd und Polyoxymethylen oder eine Mischung dieser Verbindungen.

Bei dem erfindungsgemäßen Verfahren können als Protonendonator sowohl anorganische Säuren, vorzugsweise Chlorwasserstoffsäure, als auch aromatische Monocarbonsäuren, vorzugsweise Benzoesäure, aromatische Dicarbonsäuren, vorzugsweise Phthalsäure, aromatische Polycarbonsäuren sowie aliphatische Monocarbonsäuren, vorzugsweise Methancarbonsäure, Ethancarbonsäure. Propancarbonsäure und Butancarbonsäure, aliphatische Dicarbonsäuren, vorzugsweise Malonsäure und Bernsteinsäure, und aliphatische Polycarbonsäuren sowie Kombinationen von diesen verwendet werden. Dabei können die Protonen liefernden Verbindungen sowohl stöchiometrisch als auch überstöchiometrisch oder unterstöchiometrisch eingesetzt werden. Unterstöchiometrischer Einsatz ist in allen den Fällen günstig, in denen sich die entstehenden Kupfersalze in der überschüssigen Säure lösen und damit die Aufarbeitung erschweren.

Das erfindungsgemäße Verfahren kann sowohl mit als auch ohne Zusatz eines Lösungsmittels durchgeführt werden. Zweckmäßig sind alle diejenigen organischen Lösungsmittel, die in Bezug auf die erfindungsgemäße Reaktion inert sind und einen hohen Siedepunkt besitzen, vorzugsweise Benzol, Toluol, Xylol, Dichlorbenzol, Dichlortoluol oder Decalin, insbesondere jedoch Nitrobenzol. Dabei kann der Zusatz eines Lösungsmittels, insbesondere Nitrobenzol, die Ausbeute bei Einsatz von Benzoesäure erheblich steigern.

Die Reaktionstemperatur ist nach oben lediglich durch die Stabilität der verwendeten Carbonsäuren in Anwesenheit von Kupfer limitiert. Sie kann innerhalb eines sehr weiten Bereiches von etwa 80 bis etwa 300 °C, vorzugsweise 140 bis 240 °C, liegen. Die erfindungsgemäße Umsetzung kann bei Atmosphärendruck oder bei Überdruck im Autoklaven durchgeführt werden.

Zu den nachfolgenden Beispielen bedeuten Prozentangaben jeweils Gewichtsprozent. Die Produkte wurden mit Hilfe von gaschromatographischen Analysen identifiziert.

### Beispiele

1) Herstellung von 4-Chlor-3-fluornitrobenzol aus 2,4-Dichlor3-fluornitrobenzol Zu einer Lösung von 630 g (2,7 Mol) 2,4-Dichlor-3-fluornitrobenzol (90 %ig; enthält 10 % 3,5-Dichlor-4-fluornitrobenzol) und 402,6 g (3,3 Mol) Benzoesäure in 600 g Nitrobenzol wurden unter Rühren bei 150 °C portionsweise 209,7 g (3,3 Mol) Kupfer-Pulver suspendiert. Nach vollständiger Zugabe wurde 4 Stunden nachgerührt, die Suspension abgesaugt, der Feststoff zweimal mit jeweils 100 g Nitrobenzol nachgewaschen und die vereinigten Mutterlaugen fraktioniert. Man erhielt 355,6 g (83 % d. Th.) 4-Chlor-3-fluornitrobenzol sowie 55 g nicht umgesetztes 3,5-Dichlor-4-fluornitrobenzol und 91 g nicht umgesetztes 2,4-Dichlor-3-fluornitrobenzol.
2) Herstellung von 4-Chlor-3-fluornitrobenzol aus 2,4-Dichlor-5-fluornitrobenzol
   a) Zu einer Lösung aus 630 g (3,0 Mol) 2,4-Dichlor-5-fluornitrobenzol und 463,6 g (3,8 Mol) Benzoesäure in 600 g Nitrobenzol wurden unter Rühren bei 150 °C portionsweise 241,5 g (3,8 Mol) Kupfer-Pulver suspendiert. Nach vollständiger Zugabe wurde 4 Stunden nachgerührt, die so erhaltene Suspension abgesaugt, der Feststoff zweimal mit jeweils 200 g Nitrobenzol nachgespült, die vereinigte Mutterlauge mit 200 g 10 %iger Natronlauge und anschließend mit 200 g Wasser gewaschen, über Natriumsulfat getrocknet und fraktioniert. Man erhielt 362,3 g (69 % d. Th.) 4-Chlor-3-fluornitrobenzol und 104 g nicht umgesetztes 2,4-Dichlor-5-fluornitrobenzol.
   b) In einem 1 I Rundkolben mit Rührer, Rückflußkühler, Innenthermometer und Tropftrichter mit Rührer wurden 420 g (2,0 Mol) 2,4-DichIor-5-fuornitrobenzol bei 150 °C vorgelegt und innerhalb einer Stunde eine Suspension aus 158,9 g (2,5 Mol) Kupferpulver in 296 g (4 Mol) Propionsäure zugetropft. Anschließend wurde 3 Stunden auf 150 °C erhitzt und die überschüssige Propionsäure abdestilliert. Die so erhaltene Suspension wurde abgesaugt, der Rückstand mit Nitrobenzol gespült und die Mutterlauge fraktioniert. Man erhielt 247,6 g (71 % d. Th.) 4-Chlor-3-fluornitrobenzol und 70,1 g nicht umgesetztes 2,4-Dichlor-5-fluornitrobenzol.
   c) In einem Glasautoklav mit Rührer und Innenthermometer wurden 52,5 g (0,25 Mol) 2,4-Dichlor-5-fluornitrobenzol und 14,5 g (0,25 Mol) Propionaldehyd vorgelegt, 4,5 g (0,07 Mol) Kupfer-Pulver suspendiert, 9,3 g (0,13 Mol) Propionsäure und 6,0 g (0,3 Mol) Wasser zugesetzt und unter Rühren 6 h auf ca. 155 °C erwärmt. Anschließend wurden alle bis 80 °C/0,06 bar flüchtigen Bestandteile abdestilliert, die so erhaltene Suspension abgesaugt und die Mutterlauge fraktioniert. Man erhielt 15,3 g (125 %, bezogen auf eingesetztes Kupfer) 4-Chlor-3-fluornitrobenzol sowie 23,1 g nicht umgesetztes 2,4-Dichlor-5-fluornitrobenzol.
   d) In einem 4 I Email-Autoklaven mit Rührer wurden 210,1 g (1,0 Mol) 2,4-Dichlor-5-fluornitrobenzol, 63,6 g (1,0 Mol) Kupfer-Pulver, 74 g (1,0 Mol) Propionsäure und 1250 g Nitrobenzol suspendiert, mit Stickstoff ein Druck von ca. 2 bar eingestellt und 5 Stunden auf 170 °C erhitzt. Nach gaschromatographischer Analyse betrug der Umsatz zu 3-Fluor-4-chlornitrobenzol nach dieser Zeit 74,3 %.
   e) In einem 4 I Email-Autoklaven mit Rührer wurden 210,1 g (1,0 Mol) 2,4-Dichlor-5-fluornitrobenzol, 63,6 g (1,0 Mol) Kupfer-Pulver, 60,1 g (1,0 Mol) Essigsäure und 1250 g Nitrobenzol suspendiert, mit Stickstoff ein Druck von ca. 2 bar eingestellt und 12 Stunden auf 170 °C erhitzt. Nach gaschromatographischer Analyse betrug der Umsatz zu 3-Fluor-4-chlornitrobenzol nach dieser Zeit 50,4 %.
   f) In einem 4 I Email-Autoklaven mit Rührer wurden 210,1 g (1,0 Mol) 2,4-Dichlor-5-fluornitrobenzol, 63,6 g (1,0 Mol) Kupfer-Pulver, 100 g (1,0 Mol) 37 %ige Salzsäure und 1250 g Nitrobenzol suspendiert, mit Stickstoff ein Druck von ca. 2 bar eingestellt und 12 Stunden auf 170 °C erhitzt. Nach gaschromatographischer Analyse betrug der Umsatz zu 3-Fluor-4-chlornitrobenzol nach dieser Zeit 22,5 %.
3) Herstellung von 3,5-Difluornitrobenzol aus 2,6-Dichlor-3,5-difluornitrobenzol Zu einer Lösung von 4,6 g (20 mmol) 2,6-Dichlor-3,5-difluornitrobenzol und 3,0 g (40 mmol) Propionsäure in 20 g Nitrobenzol gab man bei 150 °C unter Rühren portionsweise insgesamt 2,5 g (40 mmol) Kupfer-Pulver und rührte 3 Stunden bei 150 °C nach. Der Umsatz zu 3,5-Difluornitrobenzol betrug nach dieser Zeit über 60 % (GC).

## Patentansprüche

1. Verfahren zur selektiven Dehalogenierung von ortho- Halogennitrobenzolen, wobei Halogen Chlor, Brom oder eine Kombination davon bedeutet, dadurch gekennzeichnet, daß in Nitrobenzolen der allgemeinen Formel (I) 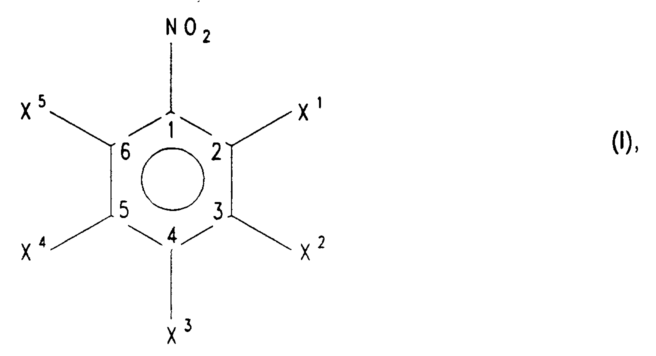 worin X¹ und X³ oder X¹ und X⁵ jeweils Chlor, Brom oder eine Kombination davon bedeuten und die jeweils übrigen Substituenten X unabhängig voneinander Wasserstoff, Fluor oder C₁-C₄-Alkyl bedeuten, durch Umsetzung mit Kupfer in Gegenwart eines Protonendonators die Halogenatome X¹ und X⁵ jeweils durch Wasserstoffatome ersetzt werden.

2. Verfahren nach Anspruch 1, dadurch gekennzeichnet, daß in der Verbindung der Formel (I) X¹ und X³ oder X¹ und X⁵ jeweils Chlor bedeuten und X² und X⁴ unabhängig voneinander Wasserstoff oder Fluor bedeuten.

3. Verfahren nach Anspruch 1 oder 2, dadurch gekennzeichnet, daß die Verbindung der Formel (I) 2,4-Dichlor-3-fluornitrobenzol oder 2,4-Dichlor-5-fluornitrobenzol oder 2,6-Dichlor-3,5-difluornitrobenzol bedeutet.

4. Verfahren nach mindestens einem der Ansprüche 1 bis 3, dadurch gekennzeichnet, daß in Nitrobenzolen der allgemeinen Formel (I) durch Umsetzung mit Kupfer und einem Reduktionsmittel, dessen Potential ausreicht, Kupfersalze zu metallischem Kupfer zu reduzieren, vorzugsweise aliphatische Aldehyde, in Gegenwart eines Protonendonators die Halogenatome X¹ und X⁵ jeweils durch Wasserstoffatome ersetzt werden.

5. Verfahren nach Anspruch 4, dadurch gekennzeichnet, daß der aliphatische Aldehyd Methanal, Ethanal, Propanal, Butanal oder eine Mischung davon ist.

6. Verfahren nach mindestens einem der Ansprüche 1 bis 5, dadurch gekennzeichnet, daß als Protonendonator eine anorganische Säure, eine aromatische Monocarbonsäure, eine aromatische Dicarbonsäure, eine aromatische Polycarbonsäure, eine aliphatische Monocarbonsäure, eine aliphatische Dicarbonsäure, eine aliphatische Polycarbonsäure oder eine Mischung davon eingesetzt wird.

7. Verfahren nach mindestens einem der Ansprüche 1 bis 6, dadurch gekennzeichnet, daß als Protonendonator Salzsäure, Benzoesäure, Phthalsäure, Essigsäure, Propionsäure, Malonsäure, Bernsteinsäure oder eine Mischung davon eingesetzt wird.

8. Verfahren nach mindestens einem der Ansprüche 1 bis 7, dadurch gekennzeichnet, daß die Umsetzung ohne Zusatz eines Lösungsmittels durchgeführt wird.

9. Verfahren nach mindestens einem der Ansprüche 1 bis 7, dadurch gekennzeichnet, daß die Umsetzung in Gegenwart eines in bezug auf die vorliegende Reaktion inerten organischen Lösungsmittels, vorzugsweise Benzol, Toluol, Xylol, Dichlorbenzol, Dichlortoluol, Decalin, Nitrobenzol oder eine Mischung davon, durchgeführt wird.

10. Verfahren nach mindestens einem der Ansprüche 1 bis 7, dadurch gekennzeichnet, daß die Umsetzung in Gegenwart von Nitrobenzol durchgeführt wird.

11. Verfahren nach mindestens einem der Ansprüche 1 bis 10, dadurch gekennzeichnet, daß der Protonendonator in unterstöchiometrischen Mengen, bezogen auf das eingesetzte ortho-Halogennitrobenzol, eingesetzt wird.
